# EUROPEAN PATENT APPLICATION

(11) **EP 3 822 627 A1**
(43) Date of publication of application: **19.05.2021**
(21) Application number: 20207531.3
(22) Date of filing: 13.11.2020
(51) Int. Cl.: G01N 27/414

(54) **BIO-FET SENSOR ARRAY WITH MATRIX CONTROLLED ON-CHIP ELECTRODE**

(30) Priority: 15.11.2019 US 201962935748 P
(71) Applicant: Helios Bioelectronics Inc., Zhubei City, 30261 (TW)
(72) Inventor: Chen, Lin-Chien, Zhubei City 302 (TW); Zhaog, Yee-Lu, Hsinchu City 300 (TW)
(74) Representative: Fuchs Patentanwälte Partnerschaft mbB

(57) **Abstract**

The present disclosure relates to a sensing system comprising a substrate, a sensor array disposed on the substrate, and a control unit electrically connected to the sensor array. The control unit is configured to activate a first sensor of the sensor array and deactivate a second sensor of the sensor array disposed adjacent to the first sensor simultaneously.

## Description

### CROSS REFERENCE TO RELATED APPLICATIONS

This patent application claims the benefit of U.S. Prov. Ser. No. 62/935,748 filed Nov. 15, 2019, the disclosure of which is hereby incorporated by reference in its entirety.

### BACKGROUND

### 1. Field of the Disclosure

The present disclosure relates to a sensor array. More specifically, the present disclosure relates to bio-FET sensor array with matrix controlled on-chip electrode.

### 2. Description of Related Art

Ion Sensitive Field Effect Transistors (ISFET) were first developed in the 1970s, as an alternative to the glass electrodes for pH and ion measurements. As technology progresses, ISFET can be used to detect a variety of properties pertaining to biomaterials, and thus can sometimes be referred to as a FET bio sensor or a bio-FET sensor. In comparison with a MOSFET, the gate electrode of a FET bio sensor is replaced by a driving electrode immersed in an aqueous solution in contact with the gate oxide. The driving electrode of a FET bio sensor is generally disposed apart from its sensing portion, and thus the distance between the driving electrode and the sensing portion may adversely affect the accuracy of the FET bio sensor. Therefore, integration of the driving electrode into the FET bio sensor is one of the problems to be solved in the field of FET bio sensors.

### SUMMARY

According to some example embodiments of the instant disclosure, a sensing system is disclosed. The sensing system comprises a substrate, a sensor array disposed on the substrate, and a control unit electrically connected to the sensor array. The control unit is configured to activate a first sensor of the sensor array and deactivate a second sensor of the sensor array disposed adjacent to the first sensor simultaneously.

According to some example embodiments of the instant disclosure, a sensing system is disclosed. The sensing system comprises a substrate, a sensor array disposed on the substrate, and a control unit electrically connected to the sensor array. The sensor array comprises a first sensing portion, a first driving electrode, a fifth sensing portion, and a fifth driving electrode, wherein the control unit is configured to activate the first sensing portion and the first driving electrode and simultaneously deactivate the fifth sensing portion and the fifth driving electrode.

According to some example embodiments of the instant disclosure, a method of controlling a sensor array is disclosed. The method comprises providing, by a control unit, a first signal to activate a first sensing portion at a first timing. The method comprises providing, by the control unit, a second signal to activate a first driving electrode at the first timing. The method comprises providing, by the control unit, a third signal to deactivate a fifth sensing portion at the first timing. The method comprises providing, by the control unit, a fourth signal to deactivate a fifth driving electrode at the first timing. Wherein the second sensing portion and the second driving electrode are disposed adjacent to the first sensing portion and the first driving electrode.

### BRIEF DESCRIPTION OF THE DRAWINGS

Aspects of the present disclosure are readily understood from the following detailed description when read with the accompanying figures. It should be noted that various features may not be drawn to scale. In fact, the dimensions of the various features may be arbitrarily increased or reduced for clarity of discussion.
FIG. 1A is a schematic view of a sensing system in accordance with some embodiments of the present disclosure.
FIG. 1B is a cross-sectional view of a portion of a sensor array in accordance with some embodiments of the present disclosure.
FIG. 2A is a schematic view of a sensor array in accordance with some embodiments of the present disclosure.
FIG. 2B is a schematic view of a sensor array in accordance with some embodiments of the present disclosure.
FIG. 3A is a schematic view of a sensor array in accordance with some embodiments of the present disclosure.
FIG. 3B is a schematic view of a sensor array in accordance with some embodiments of the present disclosure.
FIG. 3C is a schematic view of a sensor array in accordance with some embodiments of the present disclosure.
FIG. 3D is a schematic view of a sensor array in accordance with some embodiments of the present disclosure.
FIG. 4A is a schematic view of the electric fields within a sensor array in accordance with some embodiments of the present disclosure.
FIG. 4B is a schematic view of the electric fields within a sensor array in accordance with some embodiments of the present disclosure.
FIG. 5 is a cross-sectional view of a sensor in accordance with some comparative embodiments of the present disclosure.
FIG. 6 is a cross-sectional view of a sensor in accordance with some comparative embodiments of the present disclosure.
FIG. 7A is a schematic view of a sensor in accordance with some embodiments of the present disclosure.
FIG. 7B is a cross-sectional view of a sensor in accordance with some embodiments of the present disclosure.
FIG. 8 is a flow chart including operations for controlling an array of electrical components, in accordance with some embodiments of the present disclosure.

Common reference numerals are used throughout the drawings and the detailed description to indicate the same or similar elements. The present disclosure will be more apparent from the following detailed description taken in conjunction with the accompanying drawings.

### DETAILED DESCRIPTION

The following disclosure provides for many different embodiments, or examples, for implementing different features of the provided subject matter. Specific examples of components and arrangements are described below. These are, of course, merely examples and are not intended to be limiting. In the present disclosure, reference to the formation of a first feature over or on a second feature in the description that follows may include embodiments in which the first and second features are formed in direct contact, and may also include embodiments in which additional features may be formed between the first and second features, such that the first and second features may not be in direct contact. In addition, the present disclosure may repeat reference numerals and/or letters in the various examples. This repetition is for the purpose of simplicity and clarity and does not in itself dictate a relationship between the various embodiments and/or configurations discussed.

Embodiments of the present disclosure are discussed in detail below. It should be appreciated, however, that the present disclosure provides many applicable concepts that can be embodied in a wide variety of specific contexts. The specific embodiments discussed are merely illustrative and do not limit the scope of the disclosure.

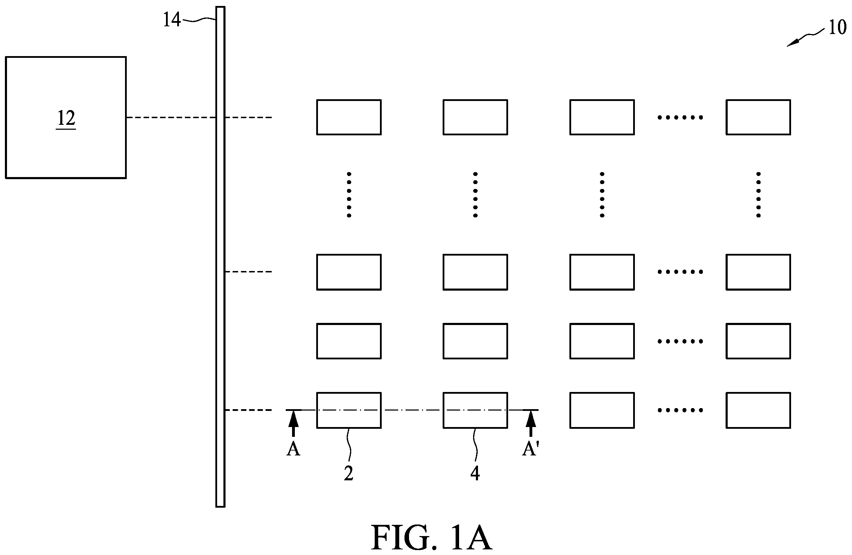

FIG. 1A is a schematic view of a sensing system in accordance with some embodiments of the present disclosure.

Referring to FIG. 1A, the sensing system includes a control unit 12, a controlling interface 14, and a sensor array 10. The control unit 12 is electrically connected to the controlling interface 14. The controlling interface 14 is electrically connected to the sensor array 10. The control unit 12 can be configured to control the operations of the sensor array 10.

In some embodiments, the control unit 12 is electrically connected to the sensor array 10 by means of the controlling interface 14. In other embodiments, the controlling interface 14 can be eliminated and thus the control unit 12 can be directly connected to the sensor array 10.

The control unit 12 may include but is not limited to, for example, a central processing unit (CPU), a microprocessor, an application-specific instruction set processor (ASIP), a machine control unit (MCU), a graphics processing unit (GPU), a physics processing unit (PPU), a digital signal processor (DSP), an image processor, a coprocessor, a storage controller, a floating-point unit, a network processor, a multi-core processor, a front-end processor or the like. In some embodiments, the control unit 12 can be a combinational logic.

In some embodiments, the controlling interface 14 can receive signals from the control unit 12 and provide the signals to one of the sensors within the sensor array 10. In other embodiments, the controlling interface 14 can relay the signals provided by the control unit 12 to a circuit (not shown) that pertains to the operations of one of the sensors within the sensor array 10. In further embodiments, the controlling interface 14 can receive signals from one of the sensors within the sensor array 10 and provide the signals to the control unit 12.

The sensor array 10 includes several sensors. The sensors of the sensor array 10 can be arranged in a matrix, and the sensor array 10 includes sensor 2 and sensor 4. In some embodiments, the sensor 2 and the sensor 4 can be bio-FET sensors, and in other embodiments, they can be sensors of other types. In some embodiments, the sensor array 10 may include sensors of the same type, and in other embodiments, the sensor array 10 may include sensors of different types.

In some embodiments, the sensor array 10 can be arranged in a rectangular profile. In some embodiments, the sensor array 10 can be arranged in a circular profile. In some embodiments, the sensor array 10 can be arranged in an irregular profile. In some embodiments, the sensor array 10 can be arranged in any suitable profile.

The sensor 2 includes a sensing portion (not shown) and a driving electrode (not shown). The sensor 4 includes a sensing portion (not shown) and a driving electrode (not shown). In some embodiments, the sensing portion and the driving electrode of the sensor 2 can be disposed adjacent to each other, while in other embodiments, they can be disposed in a substantially coplanar plane. In some embodiments, the sensing portion and the driving electrode of the sensor 4 can be disposed adjacent to each other, while in other embodiments, they can be disposed in a substantially coplanar plane.

The sensing portion and the driving electrode of the sensor 2 may be referred to as a sensor/electrode pair in the subsequent paragraphs of the present disclosure. Likewise, the sensing portion and the driving electrode of the sensor 4 may be referred to as a sensor/electrode pair in the subsequent paragraphs of the present disclosure.

A FET bio sensor can detect the amount, percentage or other properties of specific biomaterials within an aqueous solution by means of the electric field generated between the driving electrode and the sensing portion of the FET bio sensor. The electric field has influence on the current or voltage generated by the FET bio sensor, and thus the properties of a specific biomaterial can be determined by comparing the current variation or voltage variation to, for example, a look-up table generated according to experimental results. Multiple FET bio sensors disposed as an array can detect several different biomaterials within an aqueous solution at the same time. However, if multiple FET bio sensors are disposed adjacent to each other, the electric fields generated by different FET bio sensors may interfere with each other, and the interference may adversely affect the accuracy of the FET bio sensor.

For example, the electric field generated by the sensor 2 may adversely affect the accuracy of the sensor 4, and the electric field generated by the sensor 4 may adversely affect the accuracy of the sensor 2.

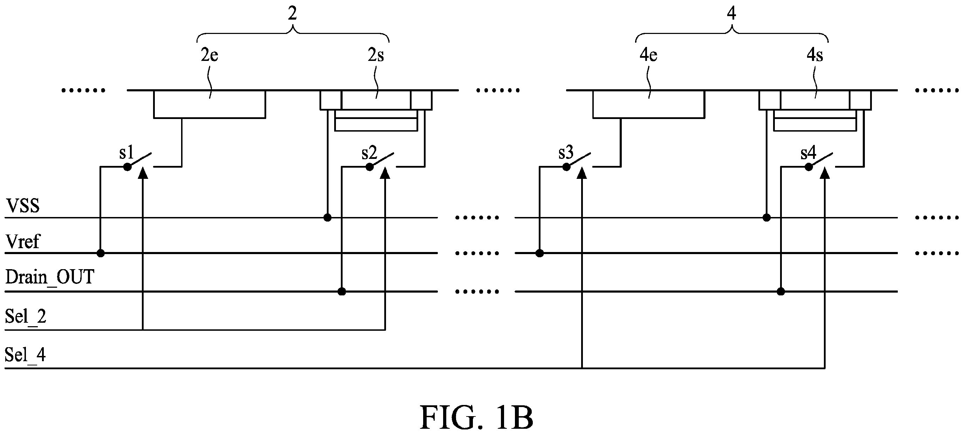

FIG. 1B is a cross-sectional view of a portion of a sensor array in accordance with some embodiments of the present disclosure. FIG. 1B shows a cross-sectional view of a portion of the sensor array 10 along the dashed-line A-A' shown in FIG. 1A. Referring to FIG. 1B, the sensor 2 includes a sensing portion 2s and a driving electrode 2e. The driving electrode 2e can be electrically connected to a reference voltage Vref through a switch s1. A signal (for example, a voltage Vref) can be provided to the driving electrode 2e when the switch s1 is turned on.

The sensing portion 2s can be electrically connected to an output terminal Drain_OUT through a switch s2. Referring to FIG. 1B, the sensor 4 includes a sensing portion 4s and a driving electrode 4e. The driving electrode 4e can be electrically connected to a reference voltage Vref through a switch s3. The sensing portion 4s can be electrically connected to an output terminal Drain_OUT through a switch s4.

The switches s1, s2, s3 and s4 can be controlled by signals provided by the control unit 12. The control unit 12 can provide signals to turn on the switches s1, s2, s3 and s4. The control unit 12 can provide signals to turn off the switches s1, s2, s3 and s4. In some embodiments, the switches s1 and s2 can be controlled by a signal Sel_2. In some embodiments, the switches s3 and s4 can be controlled by a signal Sel_4. In other embodiments, the switches s1 and s2 can be controlled by different signals. In other embodiments, the switches s3 and s4 can be controlled by different signals.

The switches s1 and s2 can be turned on simultaneously and thus the sensor 2 can be activated. The output terminal Drain_OUT can provide a signal generated by the sensing portion 2s to the control unit 12 when the switch s2 is turned on. In addition, the output terminal Drain_OUT may output a voltage generated by the sensing portion 2s, and may output a current generated by the sensing portion 2s.

The switches s3 and s4 can be turned on simultaneously and thus the sensor 4 can be activated. A signal (for example, a voltage Vref) can be provided to the driving electrode 4e when the switch s3 is turned on. The output terminal Drain_OUT can provide a signal generated by the sensing portion 4s to the control unit 12 when the switch s4 is turned on. Additionally, the output terminal Drain_OUT may output a voltage generated by the sensing portion 4s, and may output a current generated by the sensing portion 4s.

When the sensor 2 is activated, the control unit 12 can retain the sensor 4 to be inactivated. By controlling the neighboring sensor 4 to be inactivated, the interference induced by the sensor 4 can be avoided. Similarly, the control unit 12 can retain the sensor 2 to be inactivated when the sensor 4 is activated. By controlling the neighboring sensor 2 to be inactivated, the interference induced by the sensor 2 can be avoided.

In some embodiments, the sensor 2 and the sensor 4 can be activated at the same time, and in other embodiments, they can be sequentially activated. In further embodiments, the sensor 2 and the sensor 4 can be activated in accordance with the timings specified by the control unit 12.

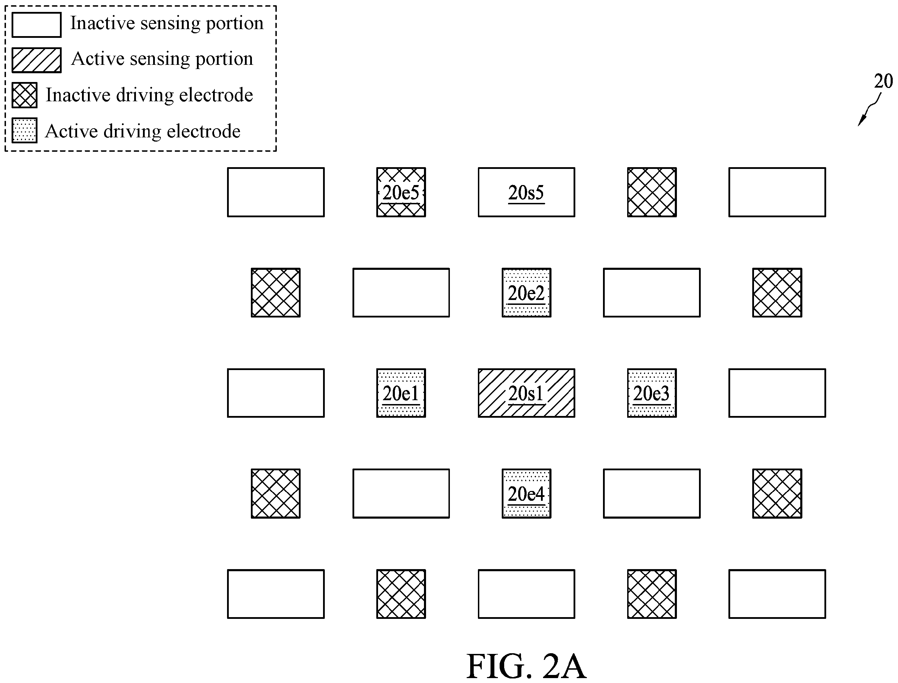

FIG. 2A is a schematic view of a sensor array in accordance with some embodiments of the present disclosure.

FIG. 2A shows an arrangement of a sensor array 20. The sensor array 20 includes a plurality of sensing portions and a plurality of driving electrodes. The sensing portions and the driving electrodes can be electrical components of different types. Each of the sensing portions can be referred to as an electrical component of a first type. Each of the driving electrodes can be referred to as an electrical component of a second type.

In some embodiments, the sensor array 20 can be disposed on a substrate. The sensor array 20 includes driving electrodes 20e1, 20e2, 20e3, 20e4 and 20e5, and sensing portions 20s1 and 20s5. The sizes of the driving electrodes and the sensing portions depicted in FIG. 2A are not drawn to scale, and are solely for illustrative purposes.

The driving electrodes 20e1, 20e2, 20e3 and 20e4 can be disposed adjacent to the sensing portion 20s1. The driving electrodes 20e1, 20e2, 20e3 and 20e4 can be disposed around the sensing portion 20s1. The driving electrode 20e1 can be disposed adjacent to a first side of the sensing portion 20s1. The driving electrode 20e2 can be disposed adjacent to a second side of the sensing portion 20s1. The driving electrode 20e3 can be disposed adjacent to a third side of the sensing portion 20s1. The driving electrode 20e4 can be disposed adjacent to a fourth side of the sensing portion 20s1.

In some embodiments, the driving electrodes 20e1, 20e2, 20e3 and 20e4 and the sensing portion 20s1 can be activated by the control unit 12 at the same time. In other embodiments, the remaining driving electrodes and the remaining sensing portions within the sensor array 20 can be deactivated by the control unit 12.

Referring to FIG. 2A, the driving electrode 20e5 can be a deactivated driving electrode during the activation period of the driving electrodes 20e1, 20e2, 20e3 and 20e4 and the sensing portion 20s1. Also referring to FIG. 2A, the sensing portion 20s5 can be a deactivated sensing portion during the activation period of the driving electrodes 20e1, 20e2, 20e3 and 20e4 and the sensing portion 20s1.

By activating the driving electrodes around the sensing portion 20s1, the electric fields sensed by the sensing portion 20s1 can be more stable. By activating the driving electrodes around the sensing portion 20s1, the signal outputted by the sensing portion 20s1 can be more accurate. Furthermore, by activating the driving electrodes around the sensing portion 20s1, the properties of biomaterials determined can be more accurate.

By deactivating the neighboring sensing portions around the sensing portion 20s1, the interference incurred by the neighboring sensing portions can be avoided, and by deactivating the driving electrodes that are not adjacent to the sensing portion 20s1, the interference incurred by those driving electrodes can also be avoided. In some embodiments, when the sensing portion 20s1 is activated, a number of N sensing portions of the sensor array 20 can be deactivated, N is a positive integer greater than one.

Referring to FIG. 2A, the sensing portion 20s1 can be the N^{th} sensing portion of the sensor array 20. The sensing portion 20s5 can be the (N+1)^{th} sensing portion of the sensor array 20. The driving electrode 20e1 can be the M^{th} driving electrode of the sensor array 20. The driving electrode 20e5 can be the (M+1)^{th} driving electrode of the sensor array 20. In some embodiments, the control unit 12 can be configured to activate the N^{th} sensing portion (i.e., sensing portion 20s1) and the M^{th} driving electrode (i.e., driving electrode 20e1) and simultaneously deactivate the (N+1)^{th} sensing portion (i.e., sensing portion 20s5) and the (M+1)^{th} driving electrode (i.e., driving electrode 20e5).

Referring to FIG. 2A, the driving electrode 20e4 can be the (M+2)^{th} driving electrode of the sensor array 20. In some embodiments, the control unit 12 can be configured to simultaneously activate the N^{th} sensing portion (i.e., sensing portion 20s1), the M^{th} driving electrode (i.e., driving electrode 20e1) and the (M+2)^{th} driving electrode (i.e., driving electrode 20e4).

Referring to FIG. 2A, the driving electrode 20e3 can be the (M+3)^{th} driving electrode of the sensor array 20, and the driving electrode 20e2 can be the (M+4)^{th} driving electrode of the sensor array 20. In some embodiments, the control unit 12 can be configured to simultaneously activate the N^{th} sensing portion (i.e., sensing portion 20s1), the M^{th} driving electrode (i.e., driving electrode 20e1), the (M+2)^{th} driving electrode (i.e., driving electrode 20e4), the (M+3)^{th} driving electrode (i.e., driving electrode 20e3) and the (M+4)^{th} driving electrode (i.e., driving electrode 20e2).

Referring to FIG. 2A,the M^{th} driving electrode (i.e., driving electrode 20e1) can be disposed adjacent to a first side of the N^{th} sensing portion (i.e., sensing portion 20s1), and the (M+3)^{th} driving electrode (i.e., driving electrode 20e3) can be disposed adjacent to a third side of the N^{th} sensing portion (i.e., sensing portion 20s1) opposite to the first side.

Referring to FIG. 2A,the (M+2)^{th} driving electrode (i.e., driving electrode 20e4) can be disposed adjacent to a second side of the N^{th} sensing portion (i.e., sensing portion 20s1), and the (M+4)^{th} driving electrode (i.e., driving electrode 20e2) can be disposed adjacent to a fourth side of the N^{th} sensing portion (i.e., sensing portion 20s1) opposite to the second side.

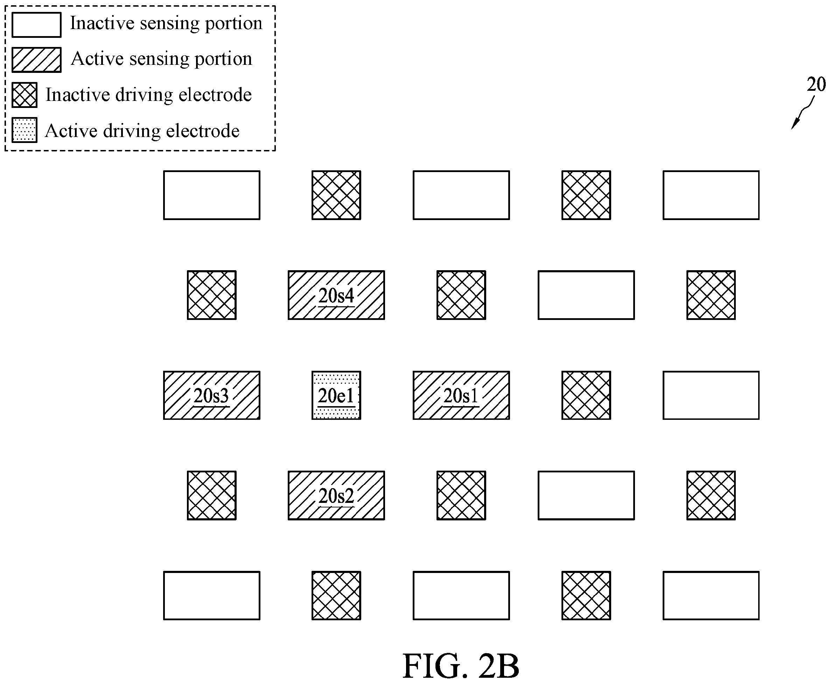

FIG. 2B is a schematic view of a sensor array in accordance with some embodiments of the present disclosure.

FIG. 2B shows an arrangement of a sensor array 20. The sensor array 20 includes a plurality of sensing portions and a plurality of driving electrodes. The sensor array 20 includes a driving electrode 20e1. The sensor array 20 includes sensing portions 20s1, 20s2, 20s3 and 20s4. The sizes of the driving electrodes and the sensing portions depicted in FIG. 2B are not drawn to scale, and are solely for illustrative purposes.

The sensing portions 20s1, 20s2, 20s3 and 20s4 can be disposed adjacent to the driving electrode 20e1. The sensing portions 20s1, 20s2, 20s3 and 20s4 can be disposed around the driving electrode 20e1 In some embodiments, the sensing portions 20s1, 20s2, 20s3 and 20s4 and the driving electrode 20e1 can be activated by the control unit 12 at the same time, and in other embodiments, the remaining driving electrodes and the remaining sensing portions within the sensor array 20 can be deactivated by the control unit 12.

By activating the sensing portions 20s1, 20s2, 20s3 and 20s4 around the driving electrode 20e1, the electric field generated by the driving electrode 20e1 can be evenly sensed by the sensing portions 20s1, 20s2, 20s3 and 20s4. By activating the sensing portions 20s1, 20s2, 20s3 and 20s4 around the driving electrode 20e1, the properties of biomaterials determined by the sensing portions 20s1, 20s2, 20s3 and 20s4 can be more accurate. In some embodiments, the activated sensing portions 20s1, 20s2, 20s3 and 20s4 can be used to detect different biomaterials properties. In some embodiments, the activated sensing portions 20s1, 20s2, 20s3 and 20s4 can provide different aspects with respect to one specific biomaterial.

Referring to FIG. 2B, the sensing portion 20s2 can be the (N+2)^{th} sensing portion of the sensor array 20. The N^{th} sensing portion (i.e., sensing portion 20s1) and the (N+2)^{th} sensing portion (i.e., sensing portion 20s2) are disposed around the M^{th} driving electrode (i.e., driving electrode 20e1). In some embodiments, the control unit 12 can be configured to simultaneously activate the N^{th} sensing portion (i.e., sensing portion 20s1), the (N+2)^{th} sensing portion (i.e., sensing portion 20s2) and the M^{th} driving electrode (i.e., driving electrode 20e1).

Referring to FIG. 2B, the sensing portion 20s3 can be the (N+3)^{th} sensing portion of the sensor array 20, and the sensing portion 20s4 can be the (N+4)^{th} sensing portion of the sensor array 20. In some embodiments, the control unit 12 can be configured to simultaneously activate the N^{th} sensing portion (i.e., sensing portion 20s1), the (N+2)^{th} sensing portion (i.e., sensing portion 20s2), the (N+3)^{th} sensing portion (i.e., sensing portion 20s3), the (N+4)^{th} sensing portion (i.e., sensing portion 20s4) and the M^{th} driving electrode (i.e., driving electrode 20e1).

Referring to FIG. 2B, the N^{th} sensing portion (i.e., sensing portion 20s1) can be disposed adjacent to a first side of the M^{th} driving electrode (i.e., driving electrode 20e1), the (N+3)^{th} sensing portion (i.e., sensing portion 20s3) can be disposed adjacent to a third side of the M^{th} driving electrode (i.e., driving electrode 20e1) opposite to the first side, the (N+2)^{th} sensing portion (i.e., sensing portion 20s2) can be disposed adjacent to a second side of the N^{th} sensing portion (i.e., sensing portion 20s1), and the (N+4)^{th} sensing portion (i.e., sensing portion 20s4) can be disposed adjacent to a fourth side of the M^{th} driving electrode (i.e., driving electrode 20e1) opposite to the second side.

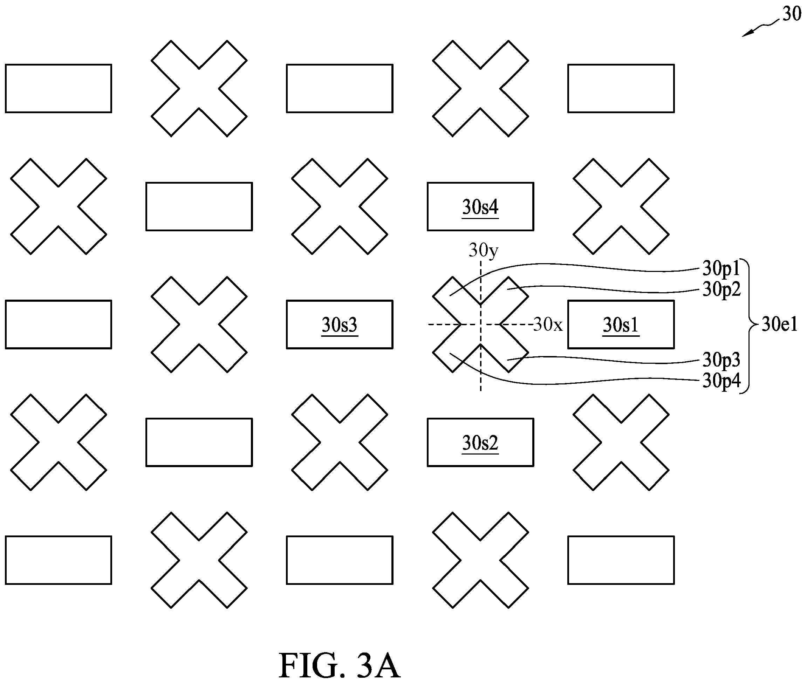

FIG. 3A is a schematic view of a sensor array in accordance with some embodiments of the present disclosure.

Referring to FIG. 3A, the sensor array 30 includes a plurality of sensing portions and a plurality of driving electrodes. In some embodiments, the sensor array 30 can be disposed on a substrate. The sensor array 30 may include driving electrode 30e1 and sensing portions 30s1, 30s2, 30s3 and 30s4. The sensing portions 30s1, 30s2, 30s3 and 30s4 can be disposed adjacent to the driving electrode 30e1. The sensing portions 30s1, 30s2, 30s3 and 30s4 can be disposed around the driving electrode 30e1.

The driving electrode 30e1 can be symmetric about an axis 30x extending in a horizontal direction. The driving electrode 30e1 can also be symmetric about an axis 30y extending in a vertical direction.

In some embodiments, the sensing portions 30s1, 30s2, 30s3 and 30s4 and the driving electrode 30e1 can be activated simultaneously by the control unit 12. In other embodiments, the remaining driving electrodes and the remaining sensing portions within the sensor array 30 can be deactivated by the control unit 12.

The driving electrode 30e1 may include a first portion 30p1, a second portion 30p2, a third portion 30p3, and a fourth portion 30p4. The first portion 30p1 may extend toward a space between the sensing portions 30s3 and 30s4. The second portion 30p2 may extend toward a space between the sensing portions 30s1 and 30s4. The third portion 30p3 may extend toward a space between the sensing portions 30s1 and 30s2. The fourth portion 30p4 may extend toward a space between the sensing portions 30s2 and 30s3.

The first portion 30p1 may enhance the accuracies of the sensing portions 30s3 and 30s4. The second portion 30p2 may enhance the accuracies of the sensing portions 30s1 and 30s4. The third portion 30p3 may enhance the accuracies of the sensing portions 30s1 and 30s2. The fourth portion 30p4 may enhance the accuracies of the sensing portions 30s2 and 30s3.

Referring to FIG. 3A, the driving electrode 30e1 can be the M^{th} driving electrode of the sensor array 30. The M^{th} driving electrode may include a first portion 30p1, a second portion 30p2, a third portion 30p3 and a fourth portion 30p4. The sensing portion 30s1 can be the N^{th} sensing portion of the sensor array30. The sensing portion 30s1 can be the N^{th} sensing portion of the sensor array30. The sensing portion 30s3 can be the (N+3)^{th} sensing portion of the sensor array30. The sensing portion 30s4 can be the (N+4)^{th} sensing portion of the sensor array30. The first portion 30p1 of the M^{th} driving electrode extends toward a space between the (N+3)^{th} sensing portion (i.e., sensing portion 30s3) and the (N+4)^{th} sensing portion (i.e., sensing portion 30s4), and the second portion 30p2 of the M^{th} driving electrode extends toward a space between the N^{th} sensing portion (i.e., sensing portion 30s1) and the (N+4)^{th} sensing portion (i.e., sensing portion 30s4).

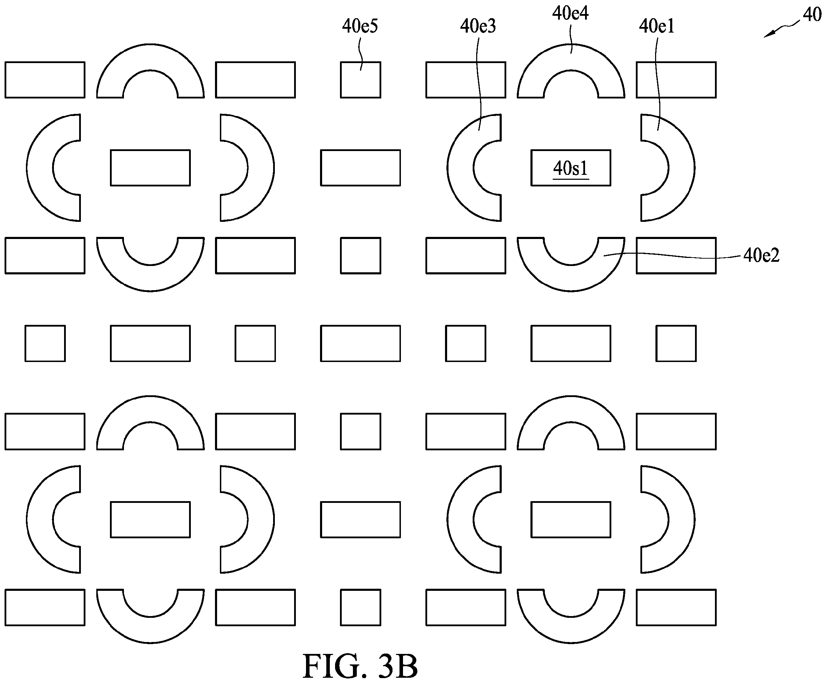

FIG. 3B is a schematic view of a sensor array in accordance with some embodiments of the present disclosure.

FIG. 3B shows an arrangement of a sensor array 40 which includes a plurality of sensing portions and a plurality of driving electrodes. In some embodiments, the sensor array 40 can be disposed on a substrate. The sensor array 40 includes driving electrodes 40e1, 40e2, 40e3, 40e4 and 40e5. In addition, the sensor array 40 includes a sensing portion 40s1. The sizes of the driving electrodes and the sensing portions depicted in FIG. 3B are not drawn to scale, and are solely for illustrative purposes.

The sensor array 40 may include driving electrodes of different shapes. In some embodiments, the shape of the driving electrode 40e1 is different from that of the driving electrode 40e5. Referring to FIG. 3B, the driving electrodes 40e1, 40e2, 40e3 and 40e4 include have shapes similar to the letter "C," and the driving electrode 40e5 has a rectangular shape. The shapes of the driving electrodes 40e1, 40e2, 40e3 and 40e4 may enhance the accuracy of the sensing portion 40s1.

The driving electrodes 40e1, 40e2, 40e3 and 40e4 can be disposed adjacent to the sensing portion 40s1. The driving electrodes 40e1, 40e2, 40e3 and 40e4 can be disposed around the sensing portion 40s1. In some embodiments, the driving electrodes 40e1, 40e2, 40e3 and 40e4 and the sensing portion 40s1 can be activated simultaneously by the control unit 12. In other embodiments, the remaining driving electrodes and the remaining sensing portions within the sensor array 40 can be deactivated by the control unit 12.

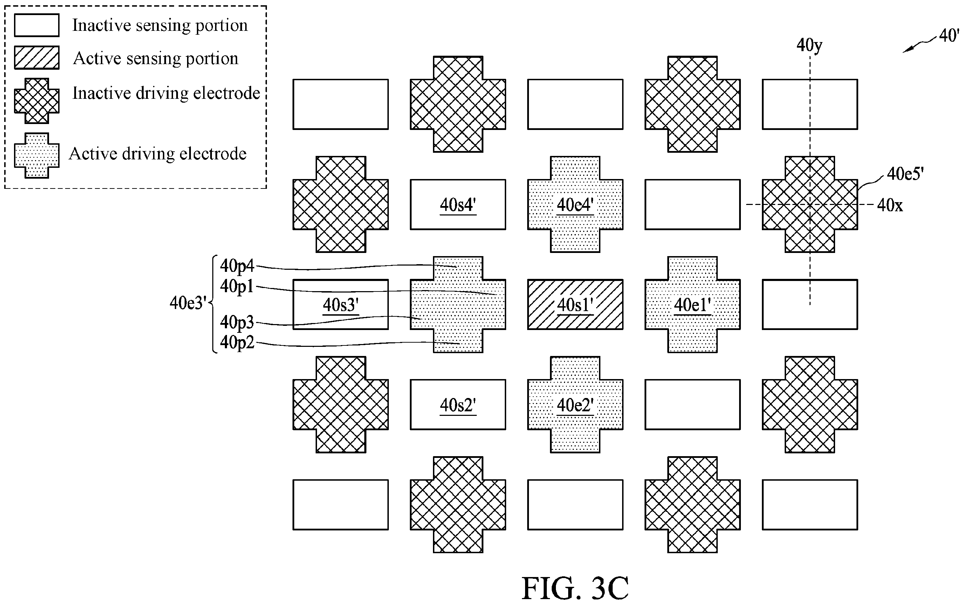

FIG. 3C is a schematic view of a sensor array in accordance with some embodiments of the present disclosure.

FIG. 3C shows an arrangement of a sensor array 40' which includes a plurality of sensing portions and a plurality of driving electrodes. In some embodiments, the sensor array 40' can be disposed on a substrate. The sensor array 40' includes driving electrodes 40e1', 40e2', 40e3', 40e4' and 40e5'. In addition, the sensor array 40' includes sensing portions 40s1', 40s2', 40s3' and 40s4'.

The driving electrodes 40e1', 40e2', 40e3' and 40e4' can be disposed adjacent to the sensing portion 40s1'. The driving electrodes 40e1', 40e2', 40e3' and 40e4' can be disposed around the sensing portion 40s1'.

In some embodiments, the driving electrodes 40e1', 40e2', 40e3' and 40e4' and the sensing portion 40s1' can be activated simultaneously by the control unit 12. In other embodiments, the remaining driving electrodes and the remaining sensing portions within the sensor array 40' can be deactivated by the control unit 12.

Referring to FIG. 3C, the driving electrode 40e5' can be symmetric about an axis 40x extending in a horizontal direction. The driving electrode 40e5' can also be symmetric about an axis 40y extending in a vertical direction. In some embodiments, the shapes of the driving electrodes 40e1', 40e2', 40e3', 40e4' and 40e5' can be substantially identical to each other.

The driving electrode 40e3' may include a first portion 40p1, a second portion 40p2, a third portion 40p3, and a fourth portion 40p4. The first portion 40p1 may extend toward the sensing portion 40s1'. The second portion 40p2 may extend toward the sensing portion 40s2'. The third portion 40p3 may extend toward the sensing portion 40s3'. The fourth portion 40p4 may extend toward the sensing portion 40s4'.

The first portion 40p1 may enhance the accuracy of the sensing portion 40s1'. The second portion 40p2 may enhance the accuracy of the sensing portion 40s2'. The third portion 40p3 may enhance the accuracy of the sensing portion 40s3'. The fourth portion 40p4 may enhance the accuracy of the sensing portion 40s4'.

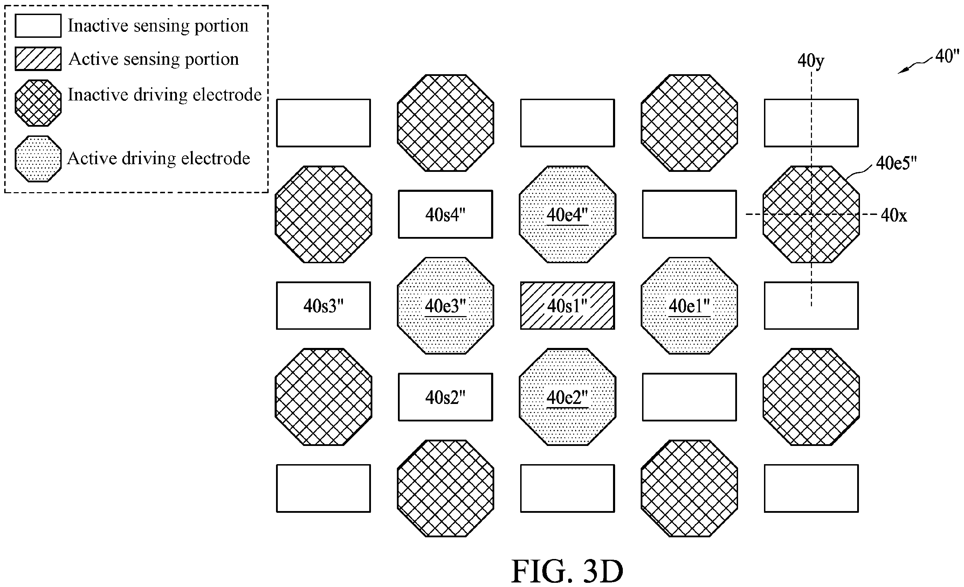

FIG. 3D is a schematic view of a sensor array in accordance with some embodiments of the present disclosure.

FIG. 3D shows an arrangement of a sensor array 40" which includes a plurality of sensing portions and a plurality of driving electrodes. In some embodiments, the sensor array 40" can be disposed on a substrate. The sensor array 40" includes driving electrodes 40e1", 40e2", 40e3", 40e4" and 40e5". In addition, the sensor array 40" includes sensing portions 40s1", 40s2", 40s3" and 40s4".

Referring to FIG. 3D, the driving electrode 40e5" can be symmetric about an axis 40x extending in a horizontal direction. The driving electrode 40e5" can also be symmetric about an axis 40y extending in a vertical direction. In some embodiments, the shapes of the driving electrodes 40e1", 40e2", 40e3", 40e4" and 40e5" can be substantially identical to each other.

The driving electrodes 40e1", 40e2", 40e3", 40e4" and 40e5" can include an octagonal shape. In some embodiments, the driving electrodes 40e1", 40e2", 40e3", 40e4" and 40e5" can be in the shape of a regular octagon. The shape of the driving electrodes 40e1", 40e2", 40e3", 40e4" and 40e5' may enhance the accuracies of their adjacent sensing portions. For example, the driving electrode 40e3" can enhance the accuracies of the sensing portions 40s1", 40s2", 40s3" and 40s4".

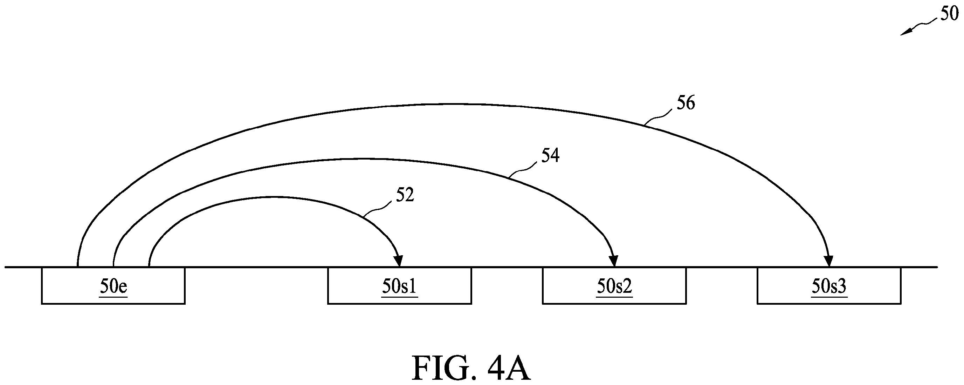

FIG. 4A is a schematic view of the electric fields within a sensor array in accordance with some embodiments of the present disclosure.

FIG. 4A shows a sensor array 50 including a driving electrode 50e and sensing portions 50s1, 50s2 and 50s3. Electric field 52 may be generated between the driving electrode 50e and the sensing portion 50s1 when the driving electrode 50e and the sensing portion 50s1 are activated. Electric field 54 may be generated between the driving electrode 50e and the sensing portion 50s2 when the driving electrode 50e and the sensing portion 50s2 are activated. Electric field 56 may be generated between the driving electrode 50e and the sensing portion 50s3 when the driving electrode 50e and the sensing portion 50s3 are activated.

The electric field 52 may interfere with the electric fields 54 or 56 and may adversely affect the accuracies of the sensing portions 50s2 and 50s3. The electric field 54 may interfere with the electric fields 52 or 56 and may adversely affect the accuracies of the sensing portions 50s1 and 50s3. The electric field 56 may interfere with the electric fields 52 or 54 and may adversely affect the accuracies of the sensing portions 50s1 and 50s2. By controlling the activations of the driving electrode 50e and a predetermined sensing portion, undesired interference can be eliminated. By activating the driving electrode 50e and a selected sensing portion while deactivating unselected sensing portions, undesired interference can be eliminated.

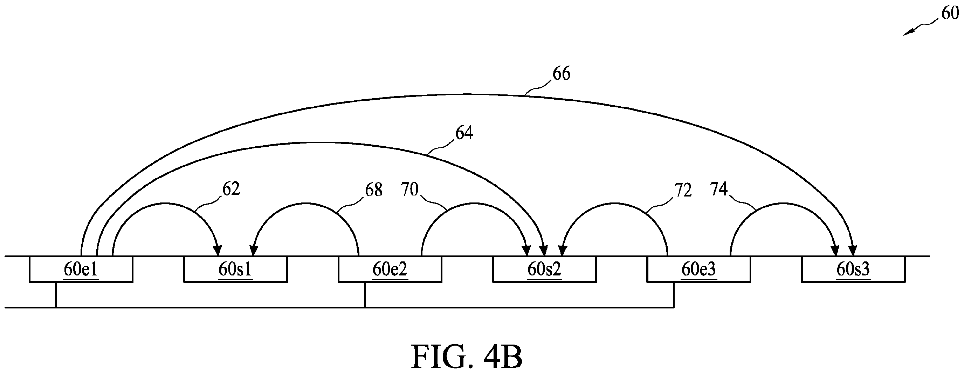

FIG. 4B is a schematic view of the electric fields within a sensor array in accordance with some embodiments of the present disclosure.

FIG. 4B shows a sensor array 60 which includes driving electrodes 60e1, 60e2 and 60e3 as well as sensing portions 60s1, 60s2 and 60s3.

Electric field 62 may be generated between the driving electrode 60e1 and the sensing portion 60s1 when they are activated. Electric field 64 may be generated between the driving electrode 60e1 and the sensing portion 60s2 when they are activated. Electric field 66 may be generated between the driving electrode 60e1 and the sensing portion 60s3 when they are activated.

Electric field 68 may be generated between the driving electrode 60e2 and the sensing portion 60s1 when they are activated. Electric field 70 may be generated between the driving electrode 60e2 and the sensing portion 60s2 when they are activated.

Electric field 72 may be generated between the driving electrode 60e3 and the sensing portion 60s2 when they are activated. Electric field 74 may be generated between the driving electrode 60e3 and the sensing portion 60s3 when they are activated.

The electric fields 62, 64, 66, 68, 70, 72 and 74 may interfere with each other. By controlling the activations of a predetermined driving electrode and a predetermined sensing portion, undesired interference can be eliminated. By activating a predetermined driving electrode and a selected sensing portion while deactivating remaining driving electrodes and sensing portions, undesired interference can be eliminated.

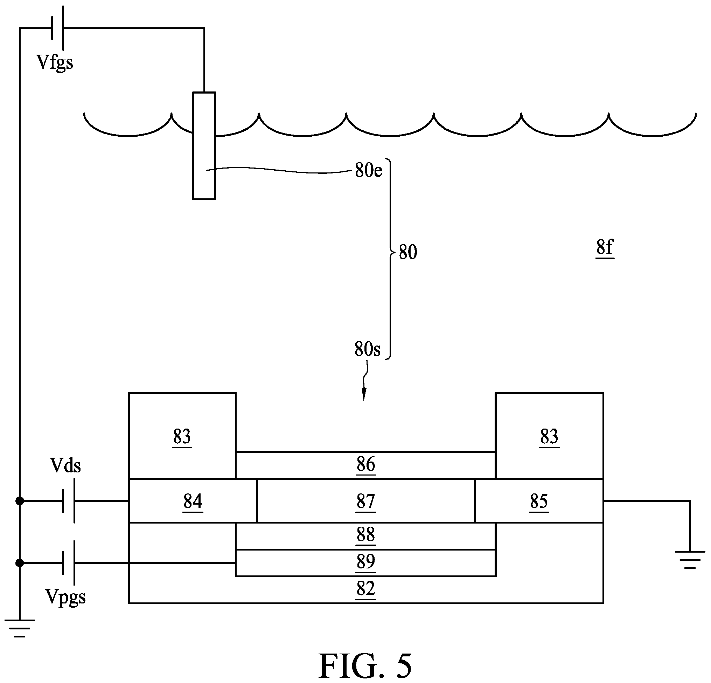

FIG. 5 is a cross-sectional view of a sensor in accordance with some comparative embodiments of the present disclosure.

Referring to FIG. 5, the sensor 80 includes a driving electrode 80e and a sensing portion 80s. The sensing portion 80s includes a substrate 82, a passivation layer 83, a drain electrode 84, a source electrode 85, an insulating layer 86, a silicon layer 87, an oxide layer 88 and a poly gate 89. The sensor 80 can be immersed into a fluid 8f. In some embodiments, the fluid 8f can be a water solution. In some embodiments, the fluid 8f can be a liquid solution. In some embodiments, the fluid 8f can be a fluid solution. In some embodiments, the source electrode 85 can be electrically connected to the ground.

A voltage Vds may exist between the drain electrode 84 and the source electrode 85, a voltage Vpgs may exist between the poly gate 89 and the source electrode 85, and a voltage Vfgs may exist between the driving electrode 80e and the source electrode 85. The voltage Vpgs can be referred to as a poly gate voltage, since it is a voltage between the poly gate 89 and the source electrode 85. The voltage Vfgs can be referred to as a fluid gate voltage, since it is a voltage between a fluid gate (i.e., the driving electrode 80e) and the source electrode 85.

The biomaterials within the fluid 8f may affect the electric fields generated by the sensor 80. The amount of a biomaterial within the fluid 8f may affect the voltage Vpgs between the poly gate 89 and the source electrode 85. The property of a biomaterial within the fluid 8f may affect the voltage Vpgs between the poly gate 89 and the source electrode 85. In some embodiments, the sensor 80 can detect the amount of a biomaterial within the fluid 8f based on the variation of the voltage Vpgs. In other embodiments, the sensor 80 can detect the property of a biomaterial within the fluid 8f based on the variation of the voltage Vpgs.

The amount of a biomaterial within the fluid 8f may affect a current Id (not shown) flowing through the drain electrode 84. The property of a biomaterial within the fluid 8f may affect a current Id flowing through the drain electrode 84. In some embodiments, the sensor 80 can detect the amount of a biomaterial within the fluid 8f based on the variation of the current Id. In certain embodiments, the sensor 80 can detect the property of a biomaterial within the fluid 8f based on the variation of the current Id.

In some embodiments, the ion or charged molecule within the fluid 8f can be attracted by the electrical fields generated between the poly gate 89 and the source electrode 85 (i.e., generated by the Vpgs), and then accumulate on the surface of the insulating layer 86. The net charge of the ion or charged molecule within the fluid 8f affects the effective Vpgs that has influence on the channel formed within the silicon layer 87, and then as a result the current Id (not shown) flowing through the drain electrode 84 may be affected.

In some embodiments, the ion or charged molecule within the fluid 8f can be attracted by the electrical fields generated between the driving electrode 80e and the sensing portion 80s (i.e., generated by the Vfgs), and then accumulate on the surface of the insulating layer 86. The net charge of the ion or charged molecule within the fluid 8f affects the effective Vpgs that has influence on the channel formed within the silicon layer 87, and then as a result the current Id (not shown) flowing through the drain electrode 84 may be affected.

Referring to FIG. 5, the driving electrode 80e is disposed spaced apart from the other portions of the sensor 80. The distance between the driving electrode 80e and the other portions of the sensor 80 may have some adverse effects on the performance of the sensor 80, including increasing the difficulty of the manufacturing of the sensor 80, bringing deviations or errors to the detection results of the sensor 80, and making it difficult to integrate the driving electrode 80e within the sensor 80. In generally, the driving electrode 80e and the sensing portion 80s are assembled manually or by equipment in different procedures. Deviations or errors can be caused by an unexpected distance between the driving electrode 80e and the sensing portion 80s since it is difficult to maintain a consistent distance if the driving electrode 80e is disposed manually or by equipment in different procedures.

Furthermore, different layers/components of the sensor 80 may include various different materials, and may have different geometric profiles, and may be formed using different procedures. These differences all make it difficult to integrate the driving electrode 80e within the sensor 80.

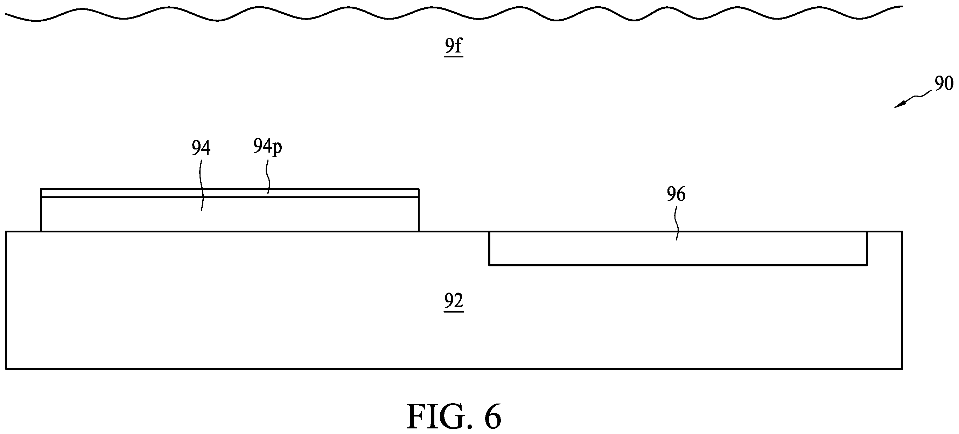

FIG. 6 is a cross-sectional view of a sensor in accordance with some comparative embodiments of the present disclosure.

Referring to FIG. 6, the sensor 90 includes a substrate 92, a driving electrode 94, and a sensor area 96. The sensor 90 can be immersed into a fluid 9f. In some embodiments, the sensor area 96 can include features and structures similar to those of the sensing portion 80s of FIG. 5.

The driving electrode 94 comprises conductive materials. In some embodiments, the driving electrode 94 comprises metal materials. In some embodiments, the driving electrode 94 may comprise alloys or multi-layer metal compond, such as Al/Si/Cu or NiTi Alloy (TiNiAg). In some embodiments, the driving electrode 94 may comprise semiconductor materials, for example silicon, one of doped silicon materials, doped polysilicon materials, or polysilicon materials. In some embodiments, the driving electrode 94 may comprise carbon (C) materials. In some embodiments, the driving electrode 94 may comprise any material that is rich in elecctron carriers.

In some embodiments, the driving electrode 94 may include precious metals. In particular embodiments, the driving electrode 94 may include one or more of gold (Au), silver (Ag), platinum (Pt), or aluminum (Al). The driving electrode 94 can be disposed near to the sensor area 96. The distance between the driving electrode 94 and the sensor area 96 can be far less than that between the driving electrode 80e and the other portions of the sensor 80. A short distance between the driving electrode 94 and the sensor area 96 make it easy to integrate the driving electrode 94 within the sensor 90. A short distance between the driving electrode 94 and the sensor area 96 may increase the accuracy of the sensor 90.

However, the driving electrode 94 made of metals or alloys may have several drawbacks. For example, the interface potential between the metals or alloys and the water solution varies depending on the types/amounts of the ion and biomaterials within the fluid. The variations resulting from the interface potential between the metals or alloys and the fluid may decrease the accuracy of the sensor 90. In another example, chemical reactions between the driving electrode 94 and the biomolecules may result in property changes (for example, activity, bonding force, etc.) to the biomolecules within the fluid.

In some embodiments, the sensor 90 may include a protection layer 94p disposed on the driving electrode 94. The protection layer 94p can include insulation materials. The protection layer 94p can include isolation materials. The protection layer 94p can prevent undesired current leakage from the driving electrode 94. The protection layer 94p can prevent undesired chemical reactions between the driving electrode 94 and the biomaterials (for example, water molecules, hydroxide ion) within the fluid 9f. In some embodiments, the protection layer 94p may indues SiO₂, Al2O₃, HfO₂, TiN, TiO₂ or mixtures thereof.

One thing should be noticed that the protection layer 94p is optional and can be eliminated if the materials of the driving electrode 94 will not have chemical reactions with water solution/fluid or the ion and biomaterials within the water solution/fluid.

In some embodiments, the protection layer 94p can be disposed on the driving electrode 94 by utilizing, for example, a coating procedure, a deposition procedure, or any other suitable procedures. In some embodiments, the protection layer 94p may include silicon dioxide. In some embodiments, the protection layer 94p can be formed through natural oxidation of the driving electrode 94.

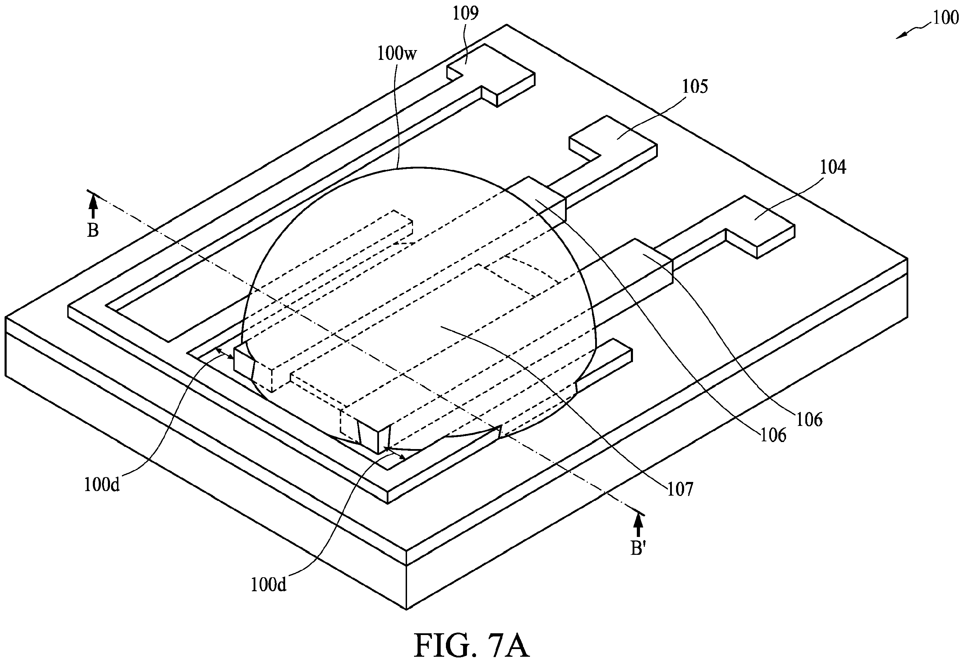

FIG. 7A is a schematic view of a sensor in accordance with some embodiments of the present disclosure.

Referring to FIG. 7A, the sensor 100 includes several electrodes disposed on a substrate. The sensor 100 includes a drain electrode 104, a source electrode 105 and a gate electrode 109. The sensor 100 includes an active silicon layer 107 disposed between the drain electrode 104 and the source electrode 105. Water solution or fluid droplet 100w can be placed above the active silicon layer 107. A distance 100d exists between the source electrode 105 and the gate electrode 109, and between the drain electrode 104 and the gate electrode 109. An insulation layer 106 can be disposed to cover a portion of the drain electrode 104, and disposed to cover a portion of the source electrode 105.

The gate electrode 109 can act as a driving electrode. The gate electrode 109 may include silicon materials. The gate electrode 109 may include doped silicon materials. In some embodiments, the gate electrode 109 may include doped P-type silicon materials. In some embodiments, the gate electrode 109 may include doped N-type silicon materials. The gate electrode 109 may include polysilicon materials. The gate electrode 109 may include doped polysilicon materials. The gate electrode 109 may include metal material with insulation materials.

The gate electrode 109 can be referred to as a poly-silicon gate or a poly gate. The sensor 100 can detect the amount/property of the biomaterials within the fluid droplet 100w. The gate electrode 109 can replace the driving electrode 94 made of metals or alloys. In addition, the gate electrode 109 can eliminate the errors resulting from the interface potential between the metals or alloys and the water solution.

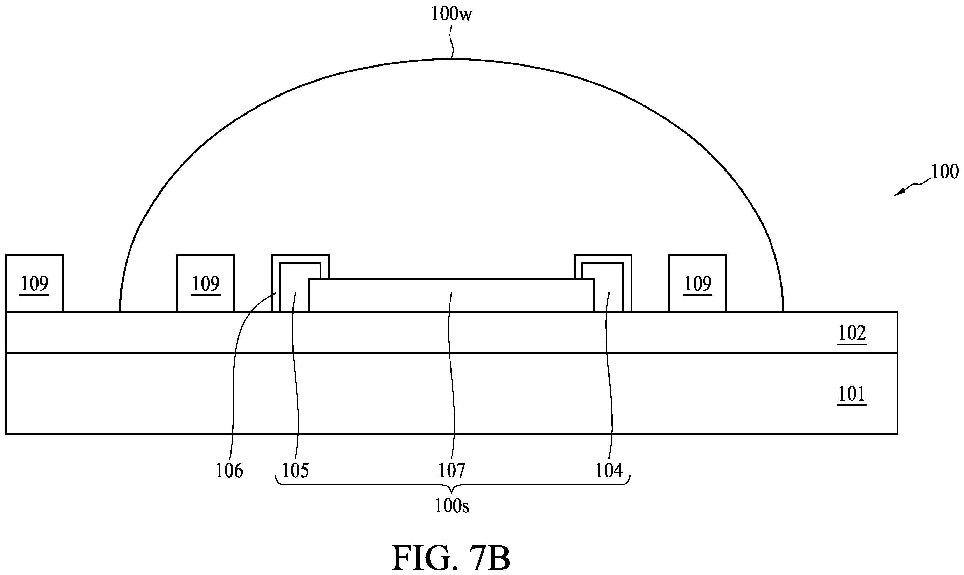

FIG. 7B is a cross-sectional view of a sensor in accordance with some embodiments of the present disclosure.

FIG. 7B shows a cross-sectional view of the sensor 100 along the dashed-line B-B' shown in FIG. 7A. The sensor 100 includes a substrate 101 and a passivation layer 102 disposed on the substrate 101. The active silicon layer 107 is disposed on the passivation layer 102. The drain electrode 104 is disposed on the passivation layer 102 and covers a portion of the active silicon layer 107. The source electrode 105 is disposed on the passivation layer 102 and covers a portion of the active silicon layer 107. The insulation layer 106 surrounds a portion of the drain electrode 104. The insulation layer 106 surrounds a portion of the source electrode 105. The sensing portion 100s and the gate electrode 109 are immersed within the fluid droplet 100w.

The sensors 2 and 4 shown in Fig. 1A may include structures similar to those of the sensor 100. The sensing portion 20s1 and the driving electrodes 20e1, 20e2, 20e3, 20e4 and 20e5 shown in Fig. 2A may include structures similar to those of the sensor 100. The sensing portion 20s1, 20s2, 20s3 and 20s4 and the driving electrode 20e1 shown in Fig. 2B may include structures similar to those of the sensor 100. The sensing portion 30s1, 30s2, 30s3 and 30s4 and the driving electrode 30e1 shown in Fig. 3B may include structures similar to those of the sensor 100. The sensing portion 40s 1 and the driving electrodes 40e1, 40e2, 40e3, 40e4 and 40e5 shown in Fig. 3B may include structures similar to those of the sensor 100.

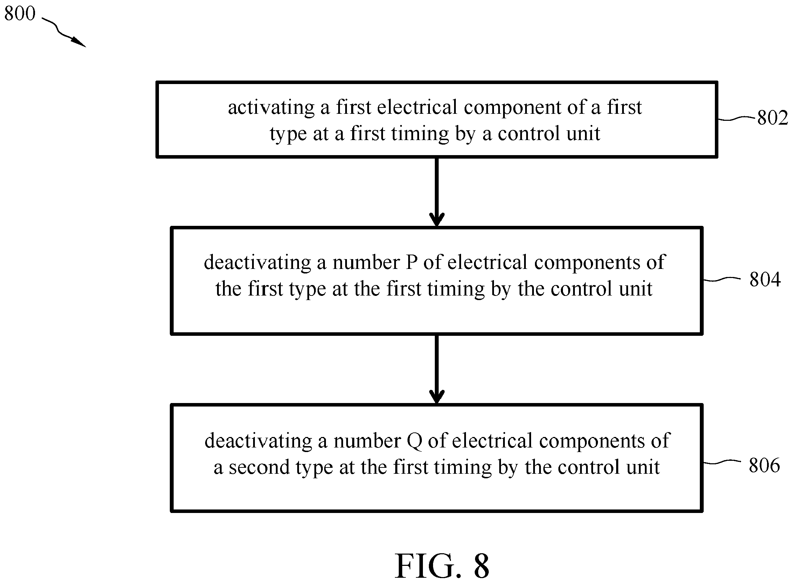

FIG. 8 is a flow chart including operations for controlling an array of electrical components, in accordance with some embodiments of the present disclosure. FIG. 8 shows a flow chart 800. The flow chart 800 includes operations 802, 804 and 806.

In the operation 802, a first electrical component of a first type can be activated at a first timing by a control unit. For example, the sensing portion 20s1 of FIG. 2A can be activated at a first timing by the control unit 12. In another example, the driving electrode 20e1 of FIG. 2B can be activated at a first timing by the control unit 12.

In the operation 804, a number P of electrical components of the first type can be deactivated at the first timing by the control unit. The number P can be an integer equal to or greater than 1. For example, referring to FIG. 2A, the sensing portion 20s5 (and all the other inactive sensing portions) can be deactivated by the control unit 12 at the first timing that the sensing portion 20s1 is activated. In another example, referring to FIG. 2B, all the inactive electrodes can be deactivated by the control unit 12 at the first timing that the driving electrode 20e1 is activated.

In the operation 806, a number Q of electrical components of a second type can be deactivated at the first timing by the control unit. The number Q can be an integer equal to or greater than 1. For example, referring to FIG. 2A, the driving electrode 20e5 (and all the other inactive driving electrodes) can be deactivated by the control unit 12 at the first timing that the sensing portion 20s1 is activated. In another example, referring to FIG. 2B, all the inactive sensors can be deactivated by the control unit 12 at the first timing that the driving electrode 20e1 is activated.

As used herein, spatially relative terms, such as "beneath," "below," "lower," "above," "upper," "lower," "left," "right" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The apparatus may be otherwise oriented (rotated 90 degrees or at other orientations) and the spatially relative descriptors used herein may likewise be interpreted accordingly. It should be understood that when an element is referred to as being "connected to" or "coupled to" another element, it may be directly connected to or coupled to the other element, or intervening elements may be present.

As used herein, the terms "approximately", "substantially", "substantial" and "about" are used to describe and account for small variations. When used in conduction with an event or circumstance, the terms can refer to instances in which the event of circumstance occurs precisely as well as instances in which the event or circumstance occurs to a close approximation. As sued herein with respect to ta given value or range, the term "about" generally means within ±10%, ±5%, ±1%, or ±0.5% of the given value or range. Ranges can be expressed herein as from one endpoint to another endpoint or between two endpoints. All ranges disclosed herein are inclusive of the endpoints, unless specified otherwise. The term "substantially coplanar" can refer to two surfaces within micrometers (µm) of lying along a same plane, such as within 10 µm, within 5 µm, within 1 µm, or within 0.5 µm of lying along the same plane. When referring to numerical values or characteristics as "substantially" the same, the term can refer to the values lying within ±10%, ±5%, ±1%, or ±0.5% of an average of the values.

The foregoing outlines features of several embodiments and detailed aspects of the present disclosure. The embodiments described in the present disclosure may be readily used as a basis for designing or modifying other processes and structures for carrying out the same or similar purposes and/or achieving the same or similar advantages of the embodiments introduced herein. Such equivalent constructions do not depart from the spirit and scope of the present disclosure, and various changes, substitutions, and alterations may be made without departing from the spirit and scope of the present disclosure.

## Claims

1. A sensing system, comprising:
a substrate;
a sensor array (10) disposed on the substrate; and
a control unit (12) for controlling the sensor array; wherein the control unit is configured to activate a first sensor (2) of the sensor array and deactivate a second sensor (4) of the sensor array disposed adjacent to the first sensor simultaneously.

2. The sensing system of Claim 1, wherein the first sensor includes a first sensing portion (2s) and a first driving electrode (2e), the first driving electrode is configured to be controlled by the control unit through a first switching circuit (s1), and the first sensing portion is configured to be controlled by the control unit through a second switching circuit (s2).

3. The sensing system of Claim 2, wherein the control unit is configured to activate the first sensor (2) by turning on the first switching circuit (s1) and the second switching circuit (s2).

4. The sensing system of Claim 1, wherein the control unit is further configured to deactivate a number of N sensors of the sensor array when the first sensor is activated, N is a positive integer greater than one.

5. The sensing system of Claim 2, wherein the driving electrode (94) comprises conductive materials, and the conductive materials comprise one of doped silicon materials, doped polysilicon materials, polysilicon materials, metal materials, alloys or carbon (C).

6. The sensing system of Claim 2, wherein the driving electrode comprises a first portion and a second portion (94p), the first portion includes the conductive materials and the second portion includes insulation materials.

7. A sensing system, comprising:
a substrate;
a sensor array (20) disposed on the substrate; and
a control unit (12) configured to control the sensor array; wherein the sensor array comprising:
a N^{th} sensing portion (20s1);
a M^{th} driving electrode (20e1);
a (N+1)^{th} sensing portion (20s5); and
a (M+1)^{th} driving electrode (20e5);
wherein the control unit is configured to activate the N^{th} sensing portion and the M^{th} driving electrode and simultaneously deactivate the (N+1)^{th} sensing portion and the (M+1)^{th} driving electrode.

8. The sensing system of Claim 7, wherein the sensor array further comprising a (M+2)^{th} driving electrode (20e4), the M^{th} driving electrode and the (M+2)^{th} driving electrode are disposed around the N^{th} sensing portion, and wherein the control unit is configured to simultaneously activate the N^{th} sensing portion, the M^{th} driving electrode and the (M+2)^{th} driving electrode.

9. The sensing system of Claim 8, wherein the sensor array further comprising a (M+3)^{th} driving electrode (20e3) and a (M+4)^{th} driving electrode (20e2) disposed around the N^{th} sensing portion, and wherein the control unit is configured to simultaneously activate the N^{th} sensing portion, the M^{th} driving electrode, the (M+2)^{th} driving electrode, the (M+3)^{th} driving electrode and the (M+4)^{th} driving electrode.

10. The sensing system of Claim 9, wherein the M^{th} driving electrode (20e1) is disposed adjacent to a first side of the N^{th} sensing portion (20s1), and the (M+3)^{th} driving electrode (20e3) is disposed adjacent to a third side of the N^{th} sensing portion opposite to the first side.

11. The sensing system of Claim 9, wherein the (M+2)^{th} driving electrode (20e4) is disposed adjacent to a second side of the N^{th} sensing portion, and the (M+4)^{th} driving electrode (20e2) is disposed adjacent to a fourth side of the N^{th} sensing portion opposite to the second side.

12. The sensing system of Claim 7, wherein the sensor array further comprising a (N+2)^{th} sensing portion (20s2), the N^{th} sensing portion and the (N+2)^{th} sensing portion are disposed around the M^{th} driving electrode, and wherein the control unit is configured to simultaneously activate the N^{th} sensing portion, the (N+2)^{th} sensing portion and the M^{th} driving electrode.

13. The sensing system of Claim 12, wherein the sensor array further comprising a (N+3)^{th} sensing portion (20s3) and a (N+4)^{th} sensing portion disposed around the M^{th} driving electrode, and wherein the control unit is configured to simultaneously activate the N^{th} sensing portion, the (N+2)^{th} sensing portion, the (N+3)^{th} sensing portion, the (N+4)^{th} sensing portion and the M^{th} driving electrode.

14. The sensing system of Claim 13, wherein the N^{th} sensing portion (20s1) is disposed adjacent to a first side of the M^{th} driving electrode (20e1), the (N+3)^{th} sensing portion (20s3) is disposed adjacent to a third side of the M^{th} driving electrode opposite to the first side, the (N+2)^{th} sensing portion (20s2) is disposed adjacent to a second side of the N^{th} sensing portion, and the (N+4)^{th} sensing portion (20s4) is disposed adjacent to a fourth side of the M^{th} driving electrode opposite to the second side.

15. A method (800) of controlling an array of electrical components, comprising:
activating a first electrical component of a first type at a first timing by a control unit (802);
deactivating a number P of electrical components of the first type at the first timing by the control unit (804);
deactivating a number Q of electrical components of a second type at the first timing by the control unit (806), wherein
the number P of electrical components of the first type and the number Q of electrical components of the second type are disposed adjacent to the first electrical component; and wherein
each of the first type of electrical components is a driving electrode and each of the second type of electrical components is a sensing portion, or
each of the first type of electrical component is a sensing portion and each of the second type of electrical component is a driving electrode.
